# EUROPEAN PATENT APPLICATION

(11) **EP 3 284 461 A1**
(43) Date of publication of application: **21.02.2018**
(21) Application number: 16780283.4
(22) Date of filing: 14.04.2016
(51) Int. Cl.: A61K 9/70, A61K 47/30, A61K 38/48, A61K 38/36, A61K 38/39, A61K 38/01, A61K 31/717

(54) **MULTIFUNCTIONAL HEMOSTATIC AGENT AND METHOD FOR PREPARING SAME**

(30) Priority: 15.04.2015 KR 20150053041
(71) Applicant: Samyang Biopharmaceuticals Corporation, Seoul 03129 (KR)
(72) Inventor: KIM, Hyun Kyoon, Daejeon 34195 (KR); KIM, Jin Su, Incheon 21335 (KR); YOON, Hye Sung, Daejeon 34049 (KR); KIM, Jun Bae, Daejeon 34049 (KR); YEO, Guw Dong, Daejeon 34048 (KR)
(74) Representative: Schrell, Andreas
(86) International application number: PCT/KR2016/003872
(87) International publication number: WO 2016/167560

(57) **Abstract**

Provided is a non-woven fabric type composite hemostatic agent, obtained by overlapping (i) at least one web of a first polymer selected from the group consisting of biodegradable polymers, oxidative regeneration cellulose, and alkali metal salt neutralizers of oxidative regeneration cellulose and (ii) at least one web of a second polymer selected from the group consisting of oxidative regeneration cellulose and alkali metal salt neutralizers thereof, followed by needle punching, compressing, and integrating.

## Description

### TECHNICAL FIELD

The present disclosure relates to a multifunctional hemostat and method for preparing the same. Specifically, the present disclosure relates to an integrated composite hemostat in the form of nonwoven fabric, obtained by overlapping, needle punching, compressing and thereby integrating (i) at least one web of a first polymer selected from the group consisting of a biodegradable polymer, oxidized regenerated cellulose and an alkali metal salt-neutralized product of oxidized regenerated cellulose and (ii) at least one web of a second polymer selected from the group consisting of oxidized regenerated cellulose and an alkali metal salt-neutralized product thereof, having hemostatic effect and antiadhesive function at the same time.

### BACKGROUND ART

A hemostat comprising oxidized regenerated cellulose (ORC), which is an absorbable fabric or knit, does not participate in *in vivo* coagulation cascade, but swells when contacting blood at a bleeding site and covers the bleeding site by acting as a "cork," provides a scaffold for clot formation, contracts tissues surrounding blood vessels and thereby arrests hemorrhage (A New Surgical Absorbable Hemostatic Agent; American Journal of Surgery, Volume 100, 439-446, September 1960).

In addition, oxidized regenerated cellulose-alkali metal salt (e.g., ORC-Na) in which reactive oxygen in carboxylic group (-COOH) in oxidized regenerated cellulose has been substituted with alkali metal (e.g., sodium (Na)) has the effect of absorbing blood superior to the conventional oxidized regenerated cellulose and thus shows improved hemostatic effect.

Meanwhile, it is known to manufacture a biodegradable polymer in the form of knit and disperse and apply an agent capable of promoting hemostasis such as thrombin, fibrinogen, carboxy methylcellulose (CMC), collagen and gelatin, etc. thereto, and thereby provide hemostatic effect. However, if thrombin and fibrinogen, etc. are directly applied to the oxidized regenerated cellulose, the regenerated cellulose may turn acidic during hemostatic action and thereby denaturing thrombin and fibrinogen, etc. To prevent such a denaturation, absorbable foaming agents such as polysaccharides must be used together.

In addition, International Publication Nos. WO 2006/044882 and WO 2007/117237 disclose a multi-layered hemostat composed of a nonwoven layer of a biodegradable polymer and a fabric or knitted layer of oxidized regenerated cellulose. This is a multi-layered hemostat prepared by separately preparing the oxidized regenerated cellulose in the form of a fabric or knit and the biodegradable polymer in the form of nonwoven fabric, and combining them. However, in the case of such a multi-layered hemostat, steps of solvent immersion and drying are required repeatedly, so that the overall process is complicated and the effect for preventing hemostasis and adhesion is not sufficient.

### DISCLOSURE OF INVENTION

### TECHNICAL PROBLEM

To solve the above problem of prior arts, an object of the present invention is to provide a multifunctional hemostat having improved hemostatic effect and antiadhesive effect at the same time and excellent dimensional stability due to the integrated structure, and preparing method thereof.

### SOLUTION TO PROBLEM

In order to resolve the above technical problem, one aspect of the present invention provides an integrated composite hemostat in the form of nonwoven fabric, obtained by overlapping, needle punching, compressing and thereby integrating (i) at least one web of a first polymer selected from the group consisting of a biodegradable polymer, oxidized regenerated cellulose and an alkali metal salt-neutralized product of oxidized regenerated cellulose and (ii) at least one web of a second polymer selected from the group consisting of oxidized regenerated cellulose and an alkali metal salt-neutralized product thereof, wherein the polymer webs of (i) and (ii) have different hemostatic effect or antiadhesive effect each other.

Another aspect of the present invention provides an integrated composite hemostat in the form of nonwoven fabric, obtained by overlapping, needle punching, compressing and thereby integrating (i) at least one web of a first polymer selected from the group consisting of a biodegradable polymer, oxidized regenerated cellulose and an alkali metal salt-neutralized product of oxidized regenerated cellulose, (ii) at least one web of a second polymer selected from the group consisting of oxidized regenerated cellulose and an alkali metal salt-neutralized product thereof and (iii) at least one web of a third polymer selected from the group consisting of oxidized regenerated cellulose and an alkali metal salt-neutralized product thereof, wherein the polymer webs of (i), (ii) and (iii) have different hemostatic effect or antiadhesive effect one another.

Yet another aspect of the present invention provides an integrated composite hemostat in the form of nonwoven fabric obtained by overlapping, needle punching, compressing and thereby integrating at least one web of a biodegradable polymer and at least one web of oxidized regenerated cellulose; at least one web of a biodegradable polymer and at least one web of an alkali metal salt-neutralized product of oxidized regenerated cellulose; at least one web of oxidized regenerated cellulose and at least one web of an alkali metal salt-neutralized product thereof; two or more oxidized regenerated celluloses or alkali metal salt-neutralized products thereof, each having different oxidation degrees; or at least one web of a biodegradable polymer, at least one web of oxidized regenerated cellulose and at least one web of an alkali metal salt-neutralized product of oxidized regenerated cellulose.

Yet another aspect of the present invention provides a method for preparing the integrated composite hemostat comprising (1) overlapping and needle punching (i) at least one web of a first polymer selected from the group consisting of a biodegradable polymer, oxidized regenerated cellulose and an alkali metal salt-neutralized product thereof and (ii) at least one web of a second polymer selected from the group consisting of oxidized regenerated cellulose and an alkali metal salt-neutralized product thereof; and (2) compressing the resulting product of (1), wherein the polymer webs of (i) and (ii) have different hemostatic effect or antiadhesive effect each other.

### BRIEF DESCRIPTION OF DRAWINGS

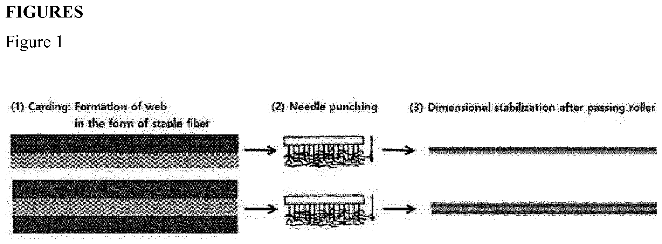

Figure 1 shows a schematic diagram of the method for preparing a hemostat according to an embodiment of the present invention.

### EFFECTS OF INVENTION

An embodiment of the present invention provides a multifunctional hemostat in the form of integrated nonwoven fabric having enhanced hemostatic and antiadhesive effect, wherein the hemostat comprises a web of biodegradable polymer staple fiber and/or ORC-neutralized product staple fiber to shield a low pH of ORC and further comprises hemostasis-promoting agents such as collagen and gelatin, etc. as well as thrombin and fibrinogen, and the hemostasis-promoting agents are applied to the web of biodegradable polymer and/or ORC-neutralized product staple fiber by immersion or powder spreading.

The hemostat of an embodiment of the present invention has a remarkably improved hemostatic effect and antiadhesive effect at the same time when compared to conventional hemostats. Therefore, when the hemostat of an embodiment of the present invention is applied to a bleeding site, it exhibits an excellent hemostatic effect as well as an effect of preventing adhesion of a hemorrhagic region and surrounding organs or tissues, thereby promoting wound healing.

In addition, according to an embodiment of the present invention, the preparation process of the hemostat can be simplified because i) the steps of isopropanol (IPA) washing and drying in a high-temperature oven are not required for staple fiber nonwoven batt, and therefore the hemostat shows excellent dimensional stability, and ii) after needle punching, it is unnecessary to take the steps of solvent immersion and drying repeatedly.

### MODE FOR THE INVENTION

Hereinafter, the present invention is explained in more detail.

In an embodiment of the present invention, the biodegradable polymer may be any conventional polymer having biocompatibility and biodegradability, and preferably selected from the group consisting of lactic acid-glycolic acid copolymer (PGLA), polyglycolic acid (PGA), poly(1,4-dioxan-2-one) (PDO), polycaprolactone (PCL), polyglycolic acid-polycaprolactone copolymer (PGA-PCL) and mixtures thereof, but is not limited thereto.

According to an embodiment of the present invention, lactic acid-glycolic acid copolymer (PGLA), polyglycolic acid (PGA), poly(1,4-dioxan-2-one) (PDO), polycaprolactone (PCL), polyglycolic acid-polycaprolactone copolymer (PGA-PCL) or mixtures thereof may be used as the biodegradable polymer.

Preferably, the biodegradable polymer may be gamma-irradiated (e.g., Cobalt 50 radiation) so that the absorption time in the body is shortened to about 2 to 4 weeks. For example, a fiber of lactic acid-glycolic acid copolymer (PGLA910) has a bioabsorption time of about 70 days, but when 50 Mrad of Cobalt 60 radiation is applied, the average absorption time is shortened to about 14 days. By using the biodegradable polymer having a reduced bioabsorption time of about 2 weeks through gamma irradiation, the hemostatic effect of the hemostat can be further enhanced.

The amount of the biodegradable polymer contained in the hemostat of an embodiment of the present invention is not particularly limited and can be appropriately adjusted depending on the desired hemostatic effect. According to a preferred embodiment of the present invention, 5 to 50 parts by weight of the biodegradable polymer may be used per 100 parts by weight of a total amount of polymers in the hemostat, but is not limited thereto.

The fabric or knit of such a polymer can be made into a fibrillar web in the form of staple fiber by carding, and the polymer web prepared thereby can be used for manufacture of the hemostat of an embodiment of the present invention. The length of the staple fiber may range from 5 to 50 mm.

In the hemostat of an embodiment of the present invention, the oxidized regenerated cellulose means oxidized regenerated cellulose having carboxyl group (-COOH), and the alkali metal salt-neutralized product thereof means a salt in which a reactive hydrogen in the carboxyl group in the oxidized regenerated cellulose is substituted with an alkali metal by treating the oxidized regenerated cellulose with alkali metal salt compound. The content of the carboxyl group in the oxidized regenerated cellulose may be 13 to 24% by weight. Examples of the alkali metal include lithium, sodium, potassium and the like, more preferably sodium or potassium, and most preferably sodium. As the alkali metal salt compound, for example, sodium acetate, potassium acetate, sodium citrate, sodium fumarate and the like can be used. In the treatment, an alkali metal salt compound of the same molar amount as the carboxyl group in the oxidized regenerated cellulose can be used. However, the present invention is not limited thereto. The conversion ratio from -COOH to -COOM (M: alkali metal) in oxidized regenerated cellulose by alkali metal salt compound treatment is preferably 30 to 70%, more preferably 40 to 60%, and most preferably about 50%. However, the present invention is not limited thereto.

According to an embodiment of the present invention, the oxidized regenerated cellulose or the alkali metal salt-neutralized product thereof having an oxidation degree of 13 to 17% is preferable in terms of enhancing antiadhesive effect. The oxidized regenerated cellulose or the alkali metal salt-neutralized product thereof having an oxidation degree of more than 17 to 24%, for example 18 to 24%, is preferable in terms of enhancing hemostatic effect. Therefore, in the hemostat of an embodiment of the present invention, it is preferable that the oxidized regenerated cellulose or the alkali metal salt-neutralized product thereof having an oxidation degree of 18 to 24% is arranged on the side contacting the bleeding site to prevent bleeding, and the oxidized regenerated cellulose or the alkali metal salt-neutralized product thereof having an oxidation degree of 13 to 17% is arranged on the opposite side to prevent organ adhesion.

There is no particular limitation on the amount of the oxidized regenerated cellulose or alkali metal salt-neutralized product thereof in the hemostat of an embodiment of the present invention, and it can be appropriately adjusted depending on the desired hemostatic effect or antiadhesive effect. According to a preferred embodiment of the present invention, 50 to 95 parts by weight of the oxidized regenerated cellulose or alkali metal salt-neutralized product thereof may be used per 100 parts by weight of a total amount of polymers in the hemostat, but is not limited thereto.

The fabric or knit of oxidized regenerated cellulose or alkali metal salt-neutralized product thereof may be manufactured to a fibrillary web in the form of staple fiber by carding, and the obtained web of oxidized regenerated cellulose or alkali metal salt-neutralized product thereof can be used in the preparation of the hemostat of an embodiment of the present invention. The length of staple fiber may be about 5 to 50 mm.

The web of biodegradable polymer, the web of alkali metal salt-neutralized product of oxidized regenerated cellulose, or the web of biodegradable polymer and the web of alkali metal salt-neutralized product of oxidized regenerated cellulose may preferably contain a hemostasis-promoting agent. The hemostasis-promoting agent may be selected from the group consisting of thrombin, fibrinogen, thromboplastin, casein kinase II, tissue factor, carboxymethyl cellulose (CMC), collagen, gelatin and combinations thereof.

According to an embodiment of the present invention, thrombin, fibrinogen or a mixture thereof may be used as the hemostasis-promoting agent. In this case, a hemostasis-promoting agent, thrombin, fibrinogen, or the like-promoting agent, is dissolved in a physiological saline or buffer, the fabric or knit of the biodegradable polymer or the alkali metal salt-neutralized product of oxidized regenerated cellulose is immersed in the solution and then dried to prepare the fabric of knit of the biodegradable polymer or the alkali metal salt-neutralized product of oxidized regenerated cellulose containing the hemostasis-promoting agent, and then the fabric or knit is carded to produce the web of biodegradable polymer or alkali metal salt-neutralized product of oxidized regenerated cellulose containing hemostasis-promoting agent.

According to another embodiment of the present invention, collagen, gelatin or a mixture thereof may be used as the hemostasis-promoting agent. In this case, the fabric or knit of the biodegradable polymer or the alkali metal salt-neutralized product of oxidized regenerated cellulose is immersed in solution of collagen, gelatin, or the like, a hemostasis-promoting agent, and then dried, or lyophilized powder of the solution of collagen, gelatin, or the like is added to the fabric or knit of the biodegradable polymer or the alkali metal salt-neutralized product of oxidized regenerated cellulose, so that the fabric of knit of the biodegradable polymer or the alkali metal salt-neutralized product of oxidized regenerated cellulose containing the hemostasis-promoting agent is prepared. Thereafter, the fabric or knit is carded to produce the web of the biodegradable polymer or the alkali metal salt-neutralized product of oxidized regenerated cellulose containing the hemostasis-promoting agent.

If the web of biodegradable polymer or alkali metal salt-neutralized product of oxidized regenerated cellulose contains the hemostasis-promoting agent, the amount of the hemostasis-promoting agent is not particularly limited and can be appropriately adjusted depending on the kind of hemostasis-promoting agent and the desired hemostatic effect.

According to an embodiment of the present invention, thrombin per unit area of the web of biodegradable polymer or alkali metal salt-neutralized product of oxidized regenerated cellulose may be present in an amount of 5 to 200 IU/cm² and more preferably 10 to 100 IU/cm², and fibrinogen may be present in an amount of 1 to 100 mg/cm² and more preferably 2 to 50 mg/cm², but is not limited thereto.

According to another embodiment of the present invention, collagen or gelatin per unit area of the web of biodegradable polymer or alkali metal salt-neutralized product of oxidized regenerated cellulose may be present in an amount of 5 to 200 mg/cm² and more preferably 10 to 150 mg/cm², but is not limited thereto.

The integrated composite hemostat of an embodiment of the present invention can be prepared by overlapping, needle punching and compressing one or more of the polymer webs of (i) and (ii) or (i), (ii) and (iii). Equipment and methods for needle punching which are commonly used for the production of nonwoven fabric can be used in the present invention without limitation. For example, the compression may be performed by using a roller. The integrated composite hemostat prepared thereby exhibits both excellent hemostatic effect and antiadhesive effect, and is also excellent in dimensional stability.

Hereinafter, the present invention is explained in more detail with the following examples. However, the protection scope of the present invention is not limited thereto.

### Examples

### Preparation Example 1: PGLA hemostat

A fabric made of PGLA910 fiber (5 x 7cm, 0.5 g), which is a biodegradable polymer whose absorption time was adjusted to 14 days by gamma irradiation, was immersed for a given time in the hemostasis-promoting agent solution prepared by dissolving thrombin and/or fibrinogen as the hemostasis-promoting agent in a buffer solution having pH 8.0 to 8.5. Thereafter, the fabric was lyophilized at a low temperature to prevent denaturation of the hemostasis-promoting agents, vacuum dried at room temperature, and filled with nitrogen gas in an aluminum pouch so as to prepare the hemostat. The amount of the hemostasis-promoting agent present per unit area of the produced hemostat is as shown in Table 1. (Sample No. 2 contained no hemostasis-promoting agent.)

### Preparation Example 2: PGLA/ORC composite hemostat

A fabric made of PGLA910 fiber was made into fibrillar web by carding, and then made into a nonwoven fabric by needle punching. The nonwoven fabric made of PGLA910 fiber (5cm x 7cm) was immersed for a given time in the hemostasis-promoting agent solution prepared by dissolving thrombin and/or fibrinogen as the hemostasis-promoting agent in a buffer solution having pH 8.0 to 8.5. Thereafter, the nonwoven fabric was lyophilized at a low temperature to prevent denaturation of the hemostasis-promoting agents, and then vacuum dried at room temperature. Meanwhile, knit of oxidized regenerated cellulose (ORC) was made into web by carding in the same manner. A nonwoven fabric made of PGLA910 fiber (5cm x 7cm, 0.5 g) and ORC fibrillar web (5cm x 7cm, 0.5 g) were overlaid and needle punched into a single nonwoven fabric, and then it was passed through a roller to form a composite hemostat. The amount of the hemostasis-promoting agent present per unit area of the produced hemostat is as shown in Table 2. (Sample No. 1 contained no hemostasis-promoting agent.)

### Preparation Example 3: PGLA/ORC composite hemostat

The solution of collagen and/or gelatin as a hemostasis-promoting agent was lyophilized in powder form and incorporated into the fabric made of PGLA910 fiber which is a biodegradable polymer. Thereafter, the fabric of PGLA910 fiber containing hemostasis-promoting agent was made into web by a carding process. Meanwhile, knit of oxidized regenerated cellulose (ORC) was made into web in the same manner. Two kinds of webs prepared thereby were overlaid and needle punched into a single nonwoven fabric, and then it was passed through a roller to form a composite hemostat. The amount of the hemostasis-promoting agent present per unit area of the produced hemostat is as shown in Table 3.

### (Sample No. 1 contained no hemostasis-promoting agent.)

### Preparation Example 4: PGLA/ORC-Na composite hemostat

A fabric made of PGLA910 fiber was made into fibrillar web by carding, and then made into a nonwoven fabric by needle punching. The nonwoven fabric made of PGLA910 fiber (5cm x 7cm, 0.5 g) was immersed for a given time in the hemostasis-promoting agent solution prepared by dissolving thrombin and/or fibrinogen as the hemostasis-promoting agent in a buffer solution having pH 8.0 to 8.5. Thereafter, the nonwoven fabric was lyophilized at a low temperature to prevent denaturation of hemostasis-promoting agents, and then vacuum dried at room temperature. Meanwhile, knit of a sodium salt-neutralized product of oxidized regenerated cellulose (ORC-Na) having oxidation degree of 20% (5cm x 7cm, 0.5 g) was made into web in the same manner. Two kinds of webs prepared thereby were overlaid and needle punched into a single nonwoven fabric, and then it was passed through a roller to form a composite hemostat. The amount of the hemostasis-promoting agent present per unit area of the produced hemostat is as shown in Table 4. (Sample No. 1 contains no hemostasis-promoting agent.)

### Experimental Example 1

Swine spleen was partially incised 15 mm in length and 3 mm in depth, and the hemostatic effects of the hemostats prepared by Preparation Examples 1 to 4 were tested. The test results are shown in the following tables.

**Table 1. PGLA hemostat of Preparation Example 1 (N=7 each)**

| Sample No. | Sample name | Thrombin (IU/cm²) | Fibrinogen (mg/cm²) | Time to hemostasis (min) |
|---|---|---|---|---|
| 1 | surgical gauze | 0 | 0 | >13 |
| 2 | PGLA910 fabric | 0 | 0 | 10.3±3.2 |
| 3 | PGLA910 fabric | 50 | 0 | 7.5±3.8 |
| 4 | PGLA910 fabric | 50 | 5 | 4.5±0.5 |
| 5 | PGLA910 fabric | 50 | 10 | 3.6±0.7 |
| 6 | PGLA910 fabric | 0 | 5 | 7.2±1.3 |
| 7 | PGLA910 fabric | 0 | 10 | 5.5±1.8 |

**Table 2. PGLA/ORC composite hemostat of Preparation Example 2 (N=7 each)**

| Sample No. | Sample name | Thrombin (IU/cm²) | Fibrinogen (mg/cm²) | Time to hemostasis (min) |
|---|---|---|---|---|
| 1 | PGLA910/ORC composite hemostat | 0 | 0 | 3.5±1.5 |
| 2 | PGLA910/ORC composite hemostat | 50 | 0 | 2.7±1.1 |
| 3 | PGLA910/ORC composite hemostat | 50 | 5 | 2.2±0.6 |
| 4 | PGLA910/ORC composite hemostat | 50 | 10 | 1.9±0.5 |
| 5 | PGLA910/ORC composite hemostat | 0 | 5 | 2.5±0.4 |
| 6 | PGLA910/ORC composite hemostat | 0 | 10 | 2.1±0.2 |

**Table 3. PGLA/ORC composite hemostat of Preparation Example 3 (N=5 each)**

| Sample No. | Sample name | Collagen (mg/cm²) | Gelatin (mg/cm²) | Time to hemostasis (min) |
|---|---|---|---|---|
| 1 | PGLA910/ORC composite hemostat | 0 | 0 | 3.7±1.6 |
| 2 | PGLA910/ORC composite hemostat | 50 | 0 | 2.8±0.8 |
| 3 | PGLA910/ORC composite hemostat | 100 | 0 | 2.7±0.7 |
| 4 | PGLA910/ORC composite hemostat | 50 | 50 | 2.3±0.6 |
| 5 | PGLA910/ORC composite hemostat | 100 | 50 | 1.5±0.5 |
| 6 | PGLA910/ORC composite hemostat | 100 | 100 | 1.2±0.2 |
| 7 | PGLA910/ORC composite hemostat | 0 | 50 | 2.8±0.6 |
| 8 | PGLA910/ORC composite hemostat | 0 | 100 | 2.6±0.4 |

**Table 4. PGLA/ORC-Na composite hemostat of Preparation Example 4 (N=7 each)**

| Sample No. | Sample name | Thrombin (IU/cm²) | Fibrinogen (mg/cm²) | Time to hemostasis (min/sec) |
|---|---|---|---|---|
| 1 | PGLA910/ORC-Na composite hemostat | 0 | 0 | 2.0±0.8 |
| 2 | PGLA910/ORC-Na composite hemostat | 50 | 0 | 1.8±0.4 |
| 3 | PGLA910/ORC-Na composite hemostat | 50 | 5 | 1.3±0.5 |
| 4 | PGLA910/ORC-Na composite hemostat | 50 | 10 | 1.1±0.3 |
| 5 | PGLA910/ORC-Na composite hemostat | 0 | 5 | 1.5±0.4 |
| 6 | PGLA910/ORC-Na composite hemostat | 0 | 10 | 1.3±0.2 |

### Preparation Example 5: PGLA/ORC-Na/ORC-Na composite hemostat

A fabric made of PGLA910 fiber containing a hemostasis-promoting agent was made into web by carding (5cm x 7cm, 0.3 g). Meanwhile, knit of a sodium salt-neutralized product of oxidized regenerated cellulose (ORC-Na) having oxidation degree of 20% (ORC-Na having oxidation degree of 20%: 5cm x 7cm, 0.3 g) was made into web in the same manner. Meanwhile, knit of a sodium salt-neutralized product of oxidized regenerated cellulose (ORC-Na) having oxidation degree of 15% (ORC-Na having oxidation degree of 15%: 5cm x 7cm, 0.3 g) was made into web in the same manner. Three kinds of webs prepared thereby were overlaid and needle punched into a single nonwoven fabric, and then it was passed through a roller to form a composite hemostat. The kinds of the hemostasis-promoting agent used for the hemostat are as shown in the footnotes of Table 5.

### Experimental Example 2

Rats were used to evaluate antiadhesive effect. The abdominal wall and the peritoneum of the abdomen were incised, and the omentum of the appendix was scraped to damage it. After the scraped appendix was returned to the abdominal cavity, a 2 cm x 2 cm lesion was made in the right abdominal wall opposite to the scraped site of the appendix. Thereafter, the scraped site of the appendix and the damaged abdominal wall were contacted directly. The composite hemostat of Preparation Example 5 was applied to the scraped site of the appendix and then the incision was closed. Ten days after the operation, the degree of adhesion was evaluated according to the Knightly Scoring System as follows.

| Score | Adhesion grading scale (Knightly Scoring System) |
|---|---|
| 0 | no adhesion |
| 1 | a single, thin, easily separable adhesion |
| 2 | less extensive but weak adhesions that stood traction poorly |
| 3 | numerous extensive visceral adhesions that involved the parietal extensions |
| 4 | numerous extensive dense adhesions that involved the adjacent mesentery, intestine and omentum, extending to the abdominal wall |

**Table 5. PGLA/ORC-Na/ORC-Na composite hemostat of Preparation Example 5 (N=7 each)**

| Degree of adhesion | Control | Sample 1 | Sample 2 |
|---|---|---|---|
| Score 0 | 0 | 5 | 6 |
| Score 1 | 0 | 1 | 1 |
| Score 2 | 1 | 1 | 0 |
| Score 3 | 1 | 0 | 0 |
| Score 4 | 5 | 0 | 0 |

| | | | |
|---|---|---|---|
| Control: Sutured without any treatment to damaged appendix Sample 1: Thrombin, fibrinogen + PGLA910/ORC20/ORC15 composite hemostat Sample 2: Collagen, gelatin + PGLA910/ORC20/ORC15 composite hemostat | | | |

### Preparation Example 6: ORC-Na/ORC composite hemostat

A knit of a sodium salt-neutralized product of oxidized regenerated cellulose (ORC-Na) having oxidation degree of 15% was made into web by carding. Meanwhile, knit of oxidized regenerated cellulose having oxidation degree of 20% was made into web in the same manner. Two kinds of webs prepared thereby were overlaid and needle punched into a single nonwoven fabric, and then it was passed through a roller to form a composite hemostat. A hemostasis-promoting agent could be contained in ORC-Na. The preparing method thereof is described below.
(1) The sodium salt-neutralized product (ORC-Na) of oxidized regenerated cellulose (ORC 15%) was made into fibrillar web (5cm x 7cm, 0.5 g). Thrombin and/or fibrinogen as the hemostasis-promoting agents were lyophilized at a low temperature to prevent denaturation of their activity, vacuum dried at room temperature, and made into the form of powder. The prepared powder was spread onto the ORC-Na fibrillar web. A knit of oxidized regenerated cellulose having oxidation degree of 20% was made into web by carding (5cm x 7cm, 0.5 g), and this web and the ORC-Na web containing the hemostasis-promoting agent were overlaid and needle punched to produce a composite hemostat made of a single nonwoven fabric.
(2) ORC-Na was made into fibrillar web (5cm x 7cm, 0.5 g). Collagen or gelatin as the hemostasis-promoting agent in the form of powder was spread onto the ORC-Na. A knit of oxidized regenerated cellulose having oxidation degree of 20% was made into web by carding (5cm x 7cm, 0.5 g), and this web and the ORC-Na web containing the hemostasis-promoting agent were overlaid and needle punched to produce a composite hemostat made of a single nonwoven fabric. The kinds and the amount of the hemostasis-promoting agent used for preparing the hemostat are as shown in Tables 6 and 7.

**Table 6. ORC-Na/ORC composite hemostat of Preparation Example 6-(1) (N=7 each)**

| Sample No. | Sample name | Thrombin (IU/cm²) | Fibrinogen (mg/cm²) | Time to hemostasis (min) |
|---|---|---|---|---|
| 1 | ORC-Na/ORC composite hemostat | 0 | 0 | 2.1±0.5 |
| 2 | ORC-Na/ORC composite hemostat | 50 | 0 | 1.7±0.4 |
| 3 | ORC-Na/ORC composite hemostat | 50 | 10 | 1.2±0.3 |
| 4 | ORC-Na/ORC composite hemostat | 0 | 10 | 1.4±0.2 |

**Table 7. ORC-Na/ORC composite hemostat of Preparation Example 6-(2) (N=7 each)**

| Sample No. | Sample name | Collagen (mg/cm²) | Gelatin (mg/cm²) | Time to hemostasis (min) |
|---|---|---|---|---|
| 1 | ORC-Na/ORC composite hemostat | 0 | 0 | 2.2±0.6 |
| 2 | ORC-Na/ORC composite hemostat | 50 | 0 | 1.6±0.3 |
| 3 | ORC-Na/ORC composite hemostat | 50 | 50 | 1.3±0.2 |
| 4 | ORC-Na/ORC composite hemostat | 0 | 50 | 1.5±0.3 |

### Preparation Example 7: ORC-Na/ORC20%/ORC15% composite hemostat

A knit of a sodium salt-neutralized product of oxidized regenerated cellulose (ORC-Na) having oxidation degree of 15% was made into web by carding (5cm x 7cm, 0.3 g). Meanwhile, knit of oxidized regenerated cellulose having oxidation degree of 20% and oxidized regenerated cellulose having oxidation degree of 15% were made into web in the same manner (each 5cm x 7cm, 0.3 g). Three kinds of webs prepared thereby were overlaid and needle punched into single nonwoven fabric, and then it was passed through a roller to form a composite hemostat. The kinds of the hemostasis-promoting agent used for preparing the hemostat are as shown in the footnotes of Table 8.

### Experimental Example 3

The degree of adhesion was evaluated using the method and the Scoring System described in Experimental Example 2, except that the composite hemostat of Preparation Example 7 was used.

**Table 8. ORC-Na/ORC20/ORC15 composite hemostat of Preparation Example 7 (N=7 each)**

| Degree of adhesion | Control | Sample 1 | Sample 2 |
|---|---|---|---|
| Score 0 | 0 | 6 | 5 |
| Score 1 | 0 | 1 | 1 |
| Score 2 | 0 | 0 | 1 |
| Score 3 | 1 | 0 | 0 |
| Score 4 | 6 | 0 | 0 |

| | | | |
|---|---|---|---|
| Control: Sutured without any treatment to damaged appendix Sample 1: Thrombin, fibrinogen + ORC-Na/ORC20/ORC15 composite hemostat Sample 2: Collagen, gelatin + ORC-Na/ORC20/ORC15 composite hemostat | | | |

## Claims

1. An integrated composite hemostat in the form of nonwoven fabric, wherein (i) at least one web of a first polymer selected from the group consisting of a biodegradable polymer, oxidized regenerated cellulose and an alkali metal salt-neutralized product of oxidized regenerated cellulose and (ii) at least one web of a second polymer selected from the group consisting of oxidized regenerated cellulose and an alkali metal salt-neutralized product thereof are integrated by overlapping, needle punching and compression,
wherein the polymer webs of (i) and (ii) have different hemostatic effect or antiadhesive effect each other.

2. The integrated composite hemostat according to Claim 1, wherein (iii) at least one web of a third polymer selected from the group consisting of oxidized regenerated cellulose and an alkali metal salt-neutralized product thereof is further integrated,
wherein the polymer webs of (i), (ii) and (iii) have different hemostatic effect or antiadhesive effect one another.

3. The integrated composite hemostat according to Claim 1, wherein
at least one web of a biodegradable polymer and at least one web of oxidized regenerated cellulose;
at least one web of a biodegradable polymer and at least one web of an alkali metal salt-neutralized product of oxidized regenerated cellulose;
at least one web of oxidized regenerated cellulose and at least one web of an alkali metal salt-neutralized product thereof;
two or more oxidized regenerated celluloses or alkali metal salt-neutralized products thereof, each having different oxidation degrees; or
at least one web of a biodegradable polymer, at least one web of oxidized regenerated cellulose and at least one web of an alkali metal salt-neutralized product of oxidized regenerated cellulose are integrated.

4. The integrated composite hemostat according to Claim 1, wherein the biodegradable polymer is selected from the group consisting of lactic acid-glycolic acid copolymer (PGLA), polyglycolic acid (PGA), poly(1,4-dioxan-2-one) (PDO), polycaprolactone (PCL), polyglycolic acid-polycaprolactone copolymer (PGA-PCL) and mixtures thereof.

5. The integrated composite hemostat according to Claim 1, wherein the biodegradable polymer is gamma-irradiated.

6. The integrated composite hemostat according to Claim 1, wherein the polymer web is a fibrillar web prepared by carding of fabric or knit of polymer.

7. The integrated composite hemostat according to Claim 1, wherein the web of a biodegradable polymer, the web of an alkali metal salt-neutralized product of oxidized regenerated cellulose, or the web of a biodegradable polymer and the web of an alkali metal salt-neutralized product of oxidized regenerated cellulose contain a hemostasis-promoting agent.

8. The integrated composite hemostat according to Claim 7, wherein the hemostasis-promoting agent is selected from the group consisting of thrombin, fibrinogen, thromboplastin, casein kinase II, tissue factor, carboxymethyl cellulose, collagen, gelatin and combinations thereof.

9. The integrated composite hemostat according to Claim 1, wherein the alkali metal salt-neutralized product of oxidized regenerated cellulose is sodium salt of the oxidized regenerated cellulose.

10. The integrated composite hemostat according to Claim 1, wherein the oxidation degree of the oxidized regenerated cellulose or the alkali metal salt-neutralized product thereof is 13 to 24%.

11. The integrated composite hemostat according to Claim 10, wherein the oxidized regenerated cellulose or the alkali metal salt-neutralized product thereof having the oxidation degree of 18 to 24% is arranged on the side of contacting bleeding site, and the oxidized regenerated cellulose or the alkali metal salt-neutralized product thereof having the oxidation degree of 13 to 17% is arranged on the opposite side.

12. A method for preparing the integrated composite hemostat in the form of nonwoven fabric according to any one of Claims 1-11, comprising:
(1) overlapping and needle punching (i) at least one web of a first polymer selected from the group consisting of a biodegradable polymer, oxidized regenerated cellulose and an alkali metal salt-neutralized product thereof and (ii) at least one web of a second polymer selected from the group consisting of oxidized regenerated cellulose and an alkali metal salt-neutralized product thereof; and
(2) compressing the resulting product of (1),
wherein the polymer webs of (i) and (ii) have different hemostatic effect or antiadhesive effect each other.

## Amended claims

### Amended claims under Art. 19.1 PCT

1. An integrated composite hemostat in the form of nonwoven fabric, wherein (i) at least one web of a first polymer selected from the group consisting of a biodegradable polymer, oxidized regenerated cellulose and an alkali metal salt-neutralized product of oxidized regenerated cellulose and (ii) at least one web of a second polymer selected from the group consisting of oxidized regenerated cellulose and an alkali metal salt-neutralized product thereof are integrated by overlapping, needle punching and compression,
wherein the polymer webs of (i) and (ii) have different hemostatic effect or antiadhesive effect each other, and wherein the oxidation degree of the oxidized regenerated cellulose or the alkali metal salt-neutralized product thereof is 13 to 24%.

2. The integrated composite hemostat according to Claim 1, wherein (iii) at least one web of a third polymer selected from the group consisting of oxidized regenerated cellulose and an alkali metal salt-neutralized product thereof is further integrated,
wherein the polymer webs of (i), (ii) and (iii) have different hemostatic effect or antiadhesive effect one another.

3. The integrated composite hemostat according to Claim 1, wherein
at least one web of a biodegradable polymer and at least one web of oxidized regenerated cellulose;
at least one web of a biodegradable polymer and at least one web of an alkali metal salt-neutralized product of oxidized regenerated cellulose;
at least one web of oxidized regenerated cellulose and at least one web of an alkali metal salt-neutralized product thereof;
two or more oxidized regenerated celluloses or alkali metal salt-neutralized products thereof, each having different oxidation degrees; or
at least one web of a biodegradable polymer, at least one web of oxidized regenerated cellulose and at least one web of an alkali metal salt-neutralized product of oxidized regenerated cellulose are integrated.

4. The integrated composite hemostat according to Claim 1, wherein the biodegradable polymer is selected from the group consisting of lactic acid-glycolic acid copolymer (PGLA), polyglycolic acid (PGA), poly(1,4-dioxan-2-one) (PDO), polycaprolactone (PCL), polyglycolic acid-polycaprolactone copolymer (PGA-PCL) and mixtures thereof.

5. The integrated composite hemostat according to Claim 1, wherein the biodegradable polymer is gamma-irradiated.

6. The integrated composite hemostat according to Claim 1, wherein the polymer web is a fibrillar web prepared by carding of fabric or knit of polymer.

7. The integrated composite hemostat according to Claim 1, wherein the web of a biodegradable polymer, the web of an alkali metal salt-neutralized product of oxidized regenerated cellulose, or the web of a biodegradable polymer and the web of an alkali metal salt-neutralized product of oxidized regenerated cellulose contain a hemostasis-promoting agent.

8. The integrated composite hemostat according to Claim 7, wherein the hemostasis-promoting agent is selected from the group consisting of thrombin, fibrinogen, thromboplastin, casein kinase II, tissue factor, carboxymethyl cellulose, collagen, gelatin and combinations thereof.

9. The integrated composite hemostat according to Claim 1, wherein the alkali metal salt-neutralized product of oxidized regenerated cellulose is sodium salt of the oxidized regenerated cellulose.

10. The integrated composite hemostat according to Claim 1, wherein the oxidized regenerated cellulose or the alkali metal salt-neutralized product thereof having the oxidation degree of 18 to 24% is arranged on the side of contacting bleeding site, and the oxidized regenerated cellulose or the alkali metal salt-neutralized product thereof having the oxidation degree of 13 to 17% is arranged on the opposite side.

11. A method for preparing the integrated composite hemostat in the form of nonwoven fabric according to any one of Claims 1-10, comprising:
(1) overlapping and needle punching (i) at least one web of a first polymer selected from the group consisting of a biodegradable polymer, oxidized regenerated cellulose and an alkali metal salt-neutralized product thereof and (ii) at least one web of a second polymer selected from the group consisting of oxidized regenerated cellulose and an alkali metal salt-neutralized product thereof; and
(2) compressing the resulting product of (1),
wherein the polymer webs of (i) and (ii) have different hemostatic effect or antiadhesive effect each other, and wherein the oxidation degree of the oxidized regenerated cellulose or the alkali metal salt-neutralized product thereof is 13 to 24%.
